# EUROPEAN PATENT SPECIFICATION

(11) **EP 1 278 530 B1**
(45) Date of publication and mention of the grant of the patent: **15.06.2005**
(21) Application number: 01943290.5
(22) Date of filing: 26.04.2001
(51) Int. Cl.: A61K 35/22, A61K 35/14, C07B 33/00, A61P 37/00, A61P 29/00

(54) **CHEMICAL MODIFICATION OF MAMMALIAN URINE**
CHEMISCHE MODIFIZIERUNG VON HARN VON SÄUGERN
MODIFICATION CHIMIQUE D'URINE DE MAMMIFERE

(30) Priority: 26.04.2000 US 199833 P; 30.05.2000 US 207286 P
(43) Date of publication of application: 29.01.2003
(60) Divisional application: 05007256.0
(73) Proprietor: Breivogel, Boris, 67059 Ludwigshafen (DE)
(72) Inventor: BREIVOGEL, Boris, 67059 Ludwigshafen (DE); KIEF, Horst, D-67059 Ludwigshafen (DE)
(74) Representative: Wallon, Alexander
(86) International application number: PCT/EP2001/004726
(87) International publication number: WO 2001/080864

(56) References cited:
- EP-A- 0 265 548
- US-B1- 6 303 154

## Description

### Field of the invention

This invention relates to methods for the chemical modification of mammal urine. The modified urine has immune modulatory effects when administered to a mammal and can be used for the treatment of immune disorders.

### Background of the invention

Mammal blood is known to consist of a cellular and a non cellular fraction. The non cellular fraction in non hemolytic blood is called plasma, a watery solution of minerals and complex biochemical synthesis products such as proteins (derived mostly from the liver and in form of immuneglobulines from cells of the immune system), carbohydrates, proteoglycans etc.

The cellular phase can be separated by centrifugation or spontaneous sedimentation of the blood cell components and yields mostly erythrocytes and thrombocytes and a smaller fraction of leukocytes. Those can be differentiated in granulocytes and lymphocytes. Newer research has turned especially to the subgroups of the lymphocytes through flow cytometry. After specific staining of surface antigens it has been possible to identify different groups of cells among these. Cells with CD4 were called T - Helper cells (inducing and increasing immune activity), CD8 Cells were called T supressor or cytotoxic cells (reducing and controlling immune activity). A further discrimination of T helper cells in Th1 and Th2 cells was possible. Th1 cells produce preferential proinflammatory cytokins like interleukin 2, interferon gamma and tumor necrosis factor. Th1 cells are used for the attack of intracellular target antigens and tumor cells by their cytotoxic activity. The action of Th2 cells is preferential anti-inflammatory, they are producing Interleukin (IL) 4, IL 10 and IL 13. Th2 cells are used for the control of extracellular antigens. By the interaction of the Th1, the Th2 and the antigen presenting cells, the effector action of the immune system is controlled. Interferon gamma enhances the production of proinflammatory substances as tumor necrosis factor, IL 1, IL 6, IL 12, oxygen radicals and NO. IL 4 is counteracting the proinflammatory activity of these substances. Different groups of diseases show typical shifts of the balance in favor of Th1 or Th2 cells. A shift in favor of the Th1 cells is considered positive for tumor patients (anti tumor activity) but noxious for patients with organ specific autoimmune diseases (like rheumatoid arthritis, uveitis, thyreoiditis). A predominance of Th2 cells will be noxious for allergic disease (Eczema), immune globulin mediated diseases and sclerodermia. This is considered as a possible explanation for the fact, that patients with active allergies and neurodermatitis are less prone for cancer. The pathologic activity and overweight of parts of the immune system in patients with allergic skin or lung disease, rheumatic and other autoimmune disease as well as many types of cancer has led to search for complex immunemodulatory agents that can rebalance the causing unbalance to influence the resulting disease.

Mammal urine or alternatively ultrafiltrate of dialysis treatments contain a spectrum of proteins and proteoglycans and other substances that pass physiologically the kidney filter. The substances are complex and differ in size and biologic function. A lot of urine substances probably have a biologic effect, but the subcutaneous administration of sterile urine of the same host mammal has not shown mentionable effects on the cells of the immune system. The same applies to the addition of sterile urine to leukocyte cultures of the same individual, no remarkable immunemodulatory effect was observed.

US 4,632,980 (Zee et al.) discloses a method for virus inactivation of blood and blood products comprising treatment with low levels of ozone. After virus inactivation the blood components are further separated and it can be used for the intended purpose.

US 4,684,521 (Edelson) discloses a method and system for externally treating blood. It comprises treatment of blood with UV radiation in the presence of a photoactive agent.

US 4,748,120 (Wiesehahn) discloses a method for the treatment of biological compositions with psoralen derivatives under irradiation conditions.
Kief discloses in EP 0265 548 a process for the production of substances that have been bactericidaly treated and/or exhibit immunemodulatory activity and the use thereof. He refers to GP 3109691 for the application of ozone for oxidation. He describes the application of an ozone-oxygen mixture under pressure on urine or blood. He discloses a process, in which blood is centrifuged and the erythrocyte and plasma fractions are separately and repeatedly treated with an oxidant (ozone/oxygen mixture) with intermediate steps of hemolysis by adding distilled water and repeated oxidation thereafter. The recommended content of Ozone is stated between 40 - 80 ng per ml of Oxygen. For particular cases the addition of a urine filtrate treated before with an oxidant is disclosed. The addition of ascorbic acid, alternative carbonyl group carriers is proposed. As well the addition of halogens is disclosed.
A dilution of 1:10 of the final treatment before therapeutic application is recommended. Treatment can be applied orally, by inhalation or local application.
In EP 0607 593 A2 Kief discloses a process to gain cell cultures with elevated contents of body own cytokines. He uses a variation of the method described in EP 0265 548 to gain patient own cytokine patterns: Full blood is exposed to ozone and cultured. The Leucocytes are separated by different measures and are again cultured. So called lysates (blood fractions of the former full blood, e.g. erythrocytes, lymphocytes etc) or treated urine of the same patient according to EP 0265 548 can be added. Finally a cytolysis of the cultured cells is induced and the final product diluted and used for therapy.

### Brief description of the invention

It has now been found that the described methods for the oxidation of blood and urine can be modified in two different directions to yield a reliable substance for the treatment of disorders of the immune system. One direction (second embodiment) is a general simplification of the process without the loss of efficacy in light and moderate immune disorders, the other (third embodiment) is a new way to gain a more efficient therapeutic product by a variated process. In one embodiment the present invention provides a method for chemical modification of mammal urine comprising the steps of
- collecting urine from a mammal,
- treating said mammal urine with a slow oxidizing agent (air) or fast oxidizing agents (a gas atmosphere of at least 90% to 100% (v/v) oxygen alternatively containing also Ozone in a container,
- removing substances with a low molecular weight to yield a modified mammal urine.

In a further embodiment the invention provides a method of treating immune disorders comprising the step of administering to a patient in need thereof modified mammal urine prepared according to the method of the invention.

In still another embodiment the present invention provides the substance being able to modulate the immune system.

### Detailed description of the invention

The present invention discloses a method for the chemical modification of mammal urine. This method comprises the step of collecting urine from mammal. It is preferred that the collection is performed in a way to enable sterile collection of the urine. This can be done by sterile cathederization of the urinary bladder or collection of midstream urine. To keep the urine sterile a sterile container should be used, e.g. a disposable sterile plastic container. Depending on the presence of non soluble components in the urine the urine can be centrifuged and the sediment may be discarded. Conventional test methods for the screening of bacterial contaminations, are e.g. sticks test on nitrite.

The collected mammal urine is now treated with an oxidizing agent at a gas atmosphere of at least about 90% (v/v) oxygen. The term "oxygen" is meant to comprise the element oxygen in atomic and molecular pure forms, especially in the form of O₂ and O₃. O₂ is the main component of the gas atmosphere. As oxidizing agents H₂O₂ and O₃ are especially preferred. H₂O₂ may be added as a diluted solution to the urine. Suitable amounts are in the range of 1 to 3 ml of a one percent solution of H₂O₂ per 100 ml of urine. The ozone (O₃) may be added to the gas atmosphere.

If ozone is used as an oxidizing agent it should be present in a concentration of about 50 to 100 µg/ml of the oxygen atmosphere. Apparatus for the generation of ozone are commercially available and are described, e.g. in US 5,052,382 to Waenwright and US 5,053,140 to Horste. It is of high importance to perform the oxidation in a gas atmosphere having a concentration of at least 90% (v/v) of O₂. The inventors of the present invention have revealed that this combination of weak oxidizing O₂ with a strong oxidizing agent allows a reliable and reproducible modification of mammal urine. It is preferred that the O₂ concentration in the gas atmosphere is as high as possible, preferably at least 95% and more preferably at least 99%. It is most preferred that the concentration of O₂ is as high as commercially available. The only relevant other gas may be ozone in the case that ozone is used as an oxidizing agent. The oxygen should be of medical grade to avoid contaminations of the urine. The oxygen atmosphere in the container is obtained by removing the air atmosphere above the urine in the container or use of an evacuated container and addition of the O₂ atmosphere. The O₂ atmosphere may have a pressure in the range of less than atmospheric pressure up to about 1 bar above atmospheric pressure. It is preferred that the pressure in the container is close to atmospheric pressure because this facilitates handling and avoids complicated mechanisms for tight sealing of the container. It is preferred to provide intimate contact between the oxygen atmosphere and the urine. A suitable method is shaking of the urine with the oxygen atmosphere to allow reaction between the urine, the oxidizing agent and the oxygen atmosphere. The concentration of the oxidizing agent may be varied in a broad range which influences certainly the reaction velocity of the oxidizing agent. Suitable concentrations of the oxidizing agent are in a range of 1 to 5 µmole per ml urine. It is obvious for a person skilled in the art that the oxidizing agent should not incorporate any toxic substances into the urine. To allow intimate mixture of the gas atmosphere with the urine it is preferred that the volume of the gas atmosphere is at least 50% of the volume of the urine. Preferably, it is in the range of 50 to 200% of the volume of the urine and more preferably of about the same volume as the urine.

After adding the oxidizing agent one or more proteases may be added. Suitable proteases comprise serine proteases such as papaine, trpysine, chymotrypsine, mixtures thereof and the like. The enzymatic activity should be in the range of 0,1 to 2 microkat per milliliter of urine. One kat is defined as the amount of an enzyme cutting one mole of peptide bonds per second. After the addition of the oxidizing agent and optionally the at least one protease the urine is incubated in the dark in a range of 2 to 18 hours at a temperature of preferably 37°C. Lower temperatures may be used but this may require increasing the amount of oxidizing agent and the amount of proteases or extending the incubation time because the reaction velocity depends on the temperature and the concentration of the reagents. Slightly higher temperatures may also be used but substantially higher temperatures may denature sensitive components of the urine and may decrease the efficiency of the modified urine.
After the incubation, substances with a low molecular weight can be removed. The term "substances with a low molecular weight" is used to define substances having a molecular weight below about 6,000 Dalton, more preferably below about 5,000 Dalton. A preferred method for the removal is the use of an ultrafiltration unit. Suitable ultrafiltration membranes are, e.g. Ultrafree-15-Biomac-5K (commercially available from Milipore Inc. The ultrafiltration can be conveniently performed with the aid of a centrifuge which speeds up the process of ultrafiltration. Ultrafiltration furthermore reduces the amount of water in the urine thus increases the concentration of the high molecular weight components. Instead of ultrafiltration other methods can be used. As suitable method is e.g. gel filtration. After the removal of the low molecular weight substances and optionally water, the modified mammal urine is preferably treated again with an oxidizing agent and a gas atmosphere as described above. This repeated treatment increases the share of the components which are modified by the method of the present invention and thus increases the effectiveness of the treatment. The obtained modified mammal urine should be stored below 4°C or may be frozen.

The modified urine of the present invention in general may be used for treating immune disorders. Allergic diseases, rheumatic diseases, autoimmune diseases and immune deficient diseases can be successfully treated or at least be improved by this treatments. Allergic diseases include e.g. Hay fever (rhinoconjunctivitis allergica, allergic asthma, neurodermitis (Eczema) etc. Rheumatic diseases include e.g. chronic polyarthritis, systemic lupus erythermatodes etc. Other autoimmune diseases include immune vasculites, immune nephritis etc. Immune deficiencies include e.g. acquired immune deficiencies, chronic virus infections. Before administration the person skilled in the art would be aware that it is necessary to check the modified urine for sterility and absence of endotoxins. Suitable tests comprise conventional medical urine in cultures, e.g. uricult. Absence of endotoxins can be checked with Limulus Amebocyte Lysate Test (BioWhittakker Inc., Walkersville, USA). The substances prepared according to the method of the invention can be administered orally, transdermally, by inhalation or parentally. It is preferred that the modified urine is prepared from the urine of the patient to be treated with the modified urine.

In a further embodiment the present invention provides substances which have an immune modulatory effect. Treating of urine from patients with, e.g. severe allergic diseases show the disappearance of two peaks and the appearance of two new peaks. This effect is not seen in healthy individuals. It is believed that these new isolated substances can be isolated and may be successfully used as immune modulatory substances.

### Experimental details

### Modification of mammal urine

200ml urine of one mammal host are sampled in a sterile plastic container in the usual medical way to obtain sterile urine. A usual stix test on nitrite is used to screen for severe bacterial contaminations. 4 portions of 50 ml are centrifuged at 20° Celsius to 2000 times gravity. The clear solution above the sediment is drawn into a sterile syringe and transferred through a sterile bacteria filter (0,2 micrometer, e.g. from Millipore) into a sterile, evacuated glass bottle of 500 ml volume. The remaining sediment is checked microscopically for bacteria. A gas atmosphere of 100 percent oxygen with an O₃ content of 75 microgram per ml O₂ is produced in a suitable apparatus and administered under constant manual shaking into the evacuated bottle through the urine until the vacuum is equalized. Alternatively instead of a mixed Ozone/Oxygen atmosphere pure oxygen can be administered after adding 2 ml of a 1% solution of H₂O₂

Subsequently, 1 ml mixed sterile enzyme solution (comprising 3 mg Papaine - equivalent 8 F.I.P.E, 1.5 mg Trypsin - equivalent 1.1 microkat, 2 mg Chymotrypsine - equivalent 10 microkat, total proteolytic activity of 64 F.I.P.E. according to Papaine method) per 20 ml urine is added into the bottle. The mixture with the oxygen ozone or pure oxygen atmosphere is incubated upright and dark at 37° Celsius for 2 - 18 hours. After incubation, aliquots of 10 ml of the treated urine are centrifuged in a concentrator filter that will preserve all particles greater 5000 Dalton. (e.g. ultrafree - 15 - biomax - 5k) at 20° Celsius at 3000 times gravity (solution "A").

This process reduces the amount of liquid by about 50%. Solution "A" is filtered through a sterile filter (millipore, 0.2 micrometer) into another clean sterile evacuated bottle. Again a gas atmosphere of 100 percent oxygen with an O₃ content of 75 microgram per ml O₂ is produced in a suitable apparatus (Alternatively instead of a mixed Ozone/Oxygen atmosphere pure oxygen can be administered after adding 2 ml of a 1% solution of H₂O₂ under constant shaking through the urine and administered under constant manual shaking into the evacuated bottle through the urine until the vacuum is equalized. 2 ml of the solution undergo a conventional medical urine culture (e.g. uricult) to prove sterility. Another sample undergoes limulus amebocyte lysate Test (Bio Whittakker Inc, Walkersville, USA) to warrant absence of endotoxines. After passing these tests, the solution can either be used for further isolation of the contained biological active substances or be used as a complex agent, to modulate the immune activity of a patient.

### Identification of process products

HPLC analysis of human urine samples before and after the above described procedures show a vanishing of two peaks with a retention time of 25.0 and 33.5 minutes in the modified urine. Two Onew peaks show up at 19.8 and 20.6 minutes post start. Both are double peaks and reproducible. A reducing treatment with dithiotreitol, to reveal disulfide bonds was not able to alter the substances that created those peaks. Control urine HPLC of healthy adults of both sexes showed the peak at 33.5 minutes but no peaks at 19.8, 20.6 and 25.0 minutes. This provides a possibility to define the successful modification of the urine.
Alternatively, the following protocol ca be followed:

### Procedure to obtain immunoactive urine products:

This method comprises the step of collecting urine from a warm-blooded species. It is preferred that the collection is performed in a way to enable sterile collection of the urine. This can be done by sterile cathederization of the urinary bladder or collection of midstream urine. To keep the urine sterile a sterile container should be used, e.g. a disposable sterile plastic container. Depending on the presence of non soluble components in the urine the urine can be centrifuged and the sediment may be discarded. Conventional test methods for the screening of bacterial contaminations, are e.g. sticks test on nitrite. The collected urine is then either exposed or not exposed to an oxidizing agent, e.g. a gas atmosphere of at least about 90% (v/v) oxygen. The term "oxygen" is meant to comprise the element oxygen in atomic and molecular pure forms, especially in the form of O₂ and O₃. O₂ is the main component of the gas atmosphere. As oxidizing agents H₂O₂ and O₃ are especially preferred. H₂O₂ may be added as a diluted solution to the urine. Suitable amounts are in the range of 1 to 3 ml of a one percent solution of H₂O₂ per 100 ml of urine. The ozone (O₃) may be added to the gas atmosphere. If ozone is used as an oxidizing agent it should be present in a concentration of about 70 to 80 µg/ml of the oxygen atmosphere. One or several proteases may or may not be added. The resulting product may or may not be incubated for 1 - 36 hours at temperatures between 0 and 41 celsius

### Clinical results

The repetitive oral or subcutaneous administration in increasing dosage of sterile 1:10000 to 1:10 dilutions in physiologic saline of the product have shown drastic remissions in children and adults with atopic eczema (neurodermatitis). Several cases have been documented, more than 12.000 patients have been treated by different variations of the described methods.

The obtained products may be either used as above cited, or can be administered to either the same individual, the same species or another species orally, transdermally or parenterally. They can be either administered undiluted or in dilutions up to 10⁻²³ by diluting them e.g with sterile saline solution, sterile water or other solutions suitable for dilution of parenteral or oral drugs. As a preservative e.g. benzyl-alcohol (e.g. 0.1%) may be added.
They should preferably be stored below 4°C or may be frozen.

## Claims

1. A method for chemical modification of mammal urine comprising the steps of
- treating mammal urine with an oxidizing agent in a container
- removing substances with a molecular weight below 5,000 Dalton to yield a modified mammal urine,
wherein the oxidizing agent is selected from a group consisting of H₂O₂, O₃, a gas atmosphere of about 90 % to 100 % (v/v) oxygen, combinations thereof and air.

2. The method according to claim 1, wherein ultrafiltration is used for removing substances with a molecular weight below 5,000 Dalton.

3. The method according to claims 1 or 2, wherein, after removing the substances with a molecular weight below 5,000 Dalton, the modified mammal urine is treated with the oxidising agent and a gas atmosphere of about 90% to 100% (v/v) oxygen in a container.

4. The method according to any of claims 1 to 3, wherein the oxidizing agent is present at a concentration of 1 to 5 micromole per milliliter urine.

5. The method according to any of claims 1 to 4, wherein the volume of the gas atmosphere is at least 50% of the volume of the urine.

6. The method according to any of claims 1 to 5, wherein the gas atmosphere has a pressure of 0 to 1 bar above atmospheric pressure.

7. The method according to any of claims 1 to 6, wherein the gas atmosphere has at least 95% (v/v) oxygen.

8. The method according to any of claims 1 to 7, wherein ultrafiltration is used for removing substances with a molecular weight below 5,000 Dalton.

9. Modified mammal urine obtainable according to any of claims 1 to 8.

10. Use of the mammal urine according to claim 9 for the manufacture of a medicament for the treatment of immune disorders.

## Patentansprüche

1. Verfahren zur chemischen Modifizierung von Säugetierurin umfassend die Schritte
- Behandeln von Säugetierurin mit einem Oxidationsmittel in einem Behälter
- Entfernen von Substanzen mit einem Molekulargewicht unter 5000 Dalton um einen modifizierten Säugetierurin zu erhalten,
wobei das Oxidationsmitel ausgewählt ist aus einer Gruppe bestehend aus H₂O₂, O₃, einer Gasatmosphäre von etwa 90 % bis 100 % (Volumen/Volumen) Sauerstoff, Kombinationen hiervon und Luft.

2. Verfahren nach Anspruch 1, wobei das Entfernen von Substanzen mit einem Molekulargewicht unter 5000 Dalton durch Ultrafiltration bewirkt wird.

3. Verfahren nach Anspruch 1 oder 2, wobei nach dem Entfernen von Substanzen mit einem Molekulargewicht unter 5000 Dalton der modifizierte Säugetierurin mit dem Oxidationsmittel und einer Gasatmosphäre von etwa 90 % bis 100 % (Volumen/Volumen) in einem Behälter behandelt wird.

4. Verfahren nach einem der Ansprüche 1 bis 3, wobei das Oxidationsmittel in einer Konzentration von 1 to 5 Micromol pro Milliliter Urin angewendet wird.

5. Verfahren nach einem der Ansprüche 1 bis 4, wobei das Volumen der Gasatmosphäre wenigstens 50% des Volumens des Urins ausmacht.

6. Verfahren nach einem der Ansprüche 1 bis 5, wobei die Gasatmosphäre einen Druck von 0 bis 1 (Volumen/Volumen) bar oberhalb Atmosphärendruck aufweist.

7. Verfahren nach einem der Ansprüche 1 bis 5, wobei die Gasatmosphäre wenistens 95% Sauerstoff aufweist.

8. Verfahren nach einem der Ansprüche 1 bis 5, wobei das Entfernen von Substanzen mit einem Molekulargewicht unter 5000 Dalton durch Ultrafiltration bewirkt wird.

9. Modifizierter Säugetierurin, erhältlich nach einem Verfahren gemäß einem der Ansprüche 1 bis 8.

10. Verwendung von Säugetierurin gemäß Anspruch 9 zur Herstellung eines Medikaments für die Behandlung von Störungen des Immunsystems.

## Revendications

1. Procédé pour la modification chimique de l'urine d'un mammifère comprenant
- le traitement de l'urine avec un réactif oxidant dans un récipient et
- l'élimination des substances avec un poids moléculaire au-dessous de 5.000 Dalton pour obtenir un urine de mammifère modifié,
où le réactif oxidant est choisi du groupe comprenant le H₂O₂, le O₃, une atmosphère gazeuse contenant de 90% à 100% (volume/volume) de l'oxygène, des mélanges desdlts réactifs et de l'air.

2. Procédé selon la revendication 1, où la méthode pour éliminer les substances avec un poids moléculaire au-dessous de 5.000 Dalton est l'ultrafiltration.

3. Procédé selon l'une ou l'autre des revendications 1 et 2, où, après l'élimination des substances avec un poids moléculaire au-dessous de 5.000 Dalton, l'urine de mammifère modifié est traité avec le réactif oxidant et une atmosphère gazeuse contenant de 90% à 100% (volume/volume) de l'oxygène dans un récipient.

4. Procédé selon l'une ou l'autre des revendications 1 à 3, où le réactif oxidant est présent dans une concentration de 1 à 5 micromole par millilitre d'urine.

5. Procédé selon l'une ou l'autre des revendications 1 à 4, où le volume de l'atmosphère gazeuse est au moins 50% du volume de l'urine.

6. Procédé selon l'une ou l'autre des revendications 1 à 5, où l'atmosphère gazeuse a une pression de 0 à 1 bar au-dessus de la pression atmosphérique.

7. Procédé selon l'une ou l'autre des revendications 1 à 6, où l'atmosphère gazeuse contient au moins 95% (volume/volume) de l'oxygène.

8. Procédé selon l'une ou l'autre des revendications 1 à 7, où l'ultrafiltration est utilisée pour éliminer des substances avec un poids moléculaire au-dessous de 5.000 Dalton.

9. Urine de mammifère modifié obtenu selon l'une ou l'autre des revendications 1 à 8.

10. Utilisation de l'urine de mammifère selon revendication 9 pour la préparation d'un médicament pour le traitement des dysfonctions du système immunitaire.
